# EUROPEAN PATENT SPECIFICATION

(11) **EP 2 345 416 B1**
(45) Date of publication and mention of the grant of the patent: **19.06.2013**
(21) Application number: 08878080.4
(22) Date of filing: 28.11.2008
(51) Int. Cl.: A61K 36/482, A61K 36/315, A61K 36/286, A61K 36/28, A61K 36/24, A61K 36/19, A61K 31/122, A61P 3/06, A61K 127/00, A61K 131/00, A61K 133/00

(54) **PHARMACEUTICAL COMPOSITION FOR PURIFING VEIN AND PREPARATION METHOD THEREOF**
PHARMAZEUTISCHE ZUSAMMENSETZUNG ZUR REINIGUNG VON VENEN UND HERSTELLUNGSVERFAHREN DAFÜR
COMPOSITION PHARMACEUTIQUE DE PURIFICATION DES VEINES ET SA MÉTHODE D'ÉLABORATION

(30) Priority: 14.11.2008 CN 200810172194
(43) Date of publication of application: 20.07.2011
(73) Proprietor: Du, Zhizheng, Beijing 100076 (CN); Tang, Jiankui, Beijing 100076 (CN)
(72) Inventor: Du, Zhizheng, Beijing 100076 (CN); Tang, Jiankui, Beijing 100076 (CN)
(74) Representative: BRP Renaud & Partner
(86) International application number: PCT/CN2008/073237
(87) International publication number: WO 2010/054520

(56) References cited:
- WO-A2-2007/045959
- CN-A- 1 364 505
- CN-A- 1 375 295
- CN-A- 1 586 598
- CN-A- 1 623 578
- CN-A- 101 474 243

## Description

### TECHNICAL FIELD

The present invention relates to a plant extract composition and a preparation method thereof. More particularly, the present invention relates to a plant extract composition capable of purifying blood vessels and a preparation method thereof.

### BACKGROUND ART

With the development in the economic society, there are many changes in the constitutions of people's diets, where more meat, milk, and egg products are consumed, resulting in more fat, protein, cholesterol, and the like being absorbed. However, when disturbance of lipid metabolism in the body occurs due to various reasons, "rubbish" in blood vessels, such as excessive cholesterol and triglycerides, will accumulate in the body, thus causing atherosclerosis and blockage of arteries.

In 1985, the American scholars M.S. Brown and J.L. Goldstein found that there is a high-density lipoprotein (HDL) in human body's blood, which can facilitates the breakdown of excessive cholesterol and triglycerides in the blood by the liver, reverses endothelial dysfunction in blood vessels, suppresses endothelial cell apoptosis, and prevents oxidation of a low-density lipoprotein (LDL) and aggregation of platelet. However, it remains very difficult to increase HDL in the body to a level capable of treating hyperlipidemia at present. A Romanian professor has extracted a kind of substance known as H3 from the drug procaine, which can increase HDL by about 34%. However, due to its severe toxicity, the wide use of this substance is limited.

When the blockage of blood vessels is still not serious and before no symptoms appear, it is very difficult to find that accumulation of the "rubbish" in blood vessels has started. Only when symptoms of cardiovascular and cerebrovascular diseases appear, the severity thereof can be realized; at this time, however, it has become less easy to clean up the blockage in the blood vessels. Usually, one method is to perform a surgery, such as bypass or stenting procedures, while the pain and risk of the surgery are well known; and another method is to use a drug, where for hyperlipidemia diseases, a type of drugs called statins are more frequently used at present. This type of drugs has good therapeutic effects, but also has some toxicity to the liver and muscles. Moreover, the use of drugs is undesirable and often results in poor compliance.

### SUMMARY OF THE INVENTION

An object of the present invention is to provide a novel plant extract composition functioning as a scavenger of blood vessels and a preparation method thereof.

In one embodiment of this invention, a plant extract composition functioning as a scavenger of blood vessels is mainly composed of plant raw materials and an extract, wherein the plant raw materials comprise the following components according to parts by weight: 10-30 parts of Folium Isatidis, 15-35 parts of Semen Cassiae, 10-20 parts of Flos Inulae, 15-30 parts of Flos Carthami, 10-30 parts of Flos Dendranthemae, and 20-50 parts of Folium Apocyni Veneti; and the extract comprises the following components according to parts by weight: 20-40 parts of Ginkgo flavone, 20-35 parts of emodin, 30-50 parts of crataegin, 15-55 parts of gypenoside, and 20-60 parts of Panax notoginseng saponins.

In a preferred embodiment of this invention, a plant extract composition is provided, wherein the plant raw materials comprise the following components according to parts by weight: 15-25 parts of Folium Isatidis, 20-30 parts of Semen Cassiae, 12-18 parts of Flos Inulae, 20-30 parts of Flos Carthami, 15-25 parts of Flos Dendranthemae, and 30-40 parts of Folium Apocyni Veneti; and the extract comprises the following components according to parts by weight: 25-35 parts of Ginkgo flavone, 25-35 parts of emodin, 30-40 parts of crataegin, 20-40 parts of gypenoside, and 30-50 parts of Panax notoginseng saponins.

In another preferred embodiment of this invention, in addition to the above components, the plant extract composition further comprises the following raw materials according to parts by weight: 10-30 parts of lecithin, 10-30 parts of black Auricularia, and 20-80 parts of solid acetic acid, wherein the lecithin is an extract, the solid acetic acid is extracted from plants and thus it also belongs to an extract in this invention, and black Auricularia is also classified as a plant raw material in this invention although it belongs to a fungi. The addition of lecithin, black Auricularia, and solid acetic acid may further soften blood vessels, prevent vascular sclerosis, and accelerate vascular reparation.

A dry final product, prepared from said plant extract composition by the preparation method below, can be prepared into capsule, tablet, powder, or granule, with or without the addition of suitable adjuvants or carriers.

Another object of the present invention is to provide a method for preparing the plant extract composition.

A method for preparing the plant extracts composition, comprising the following steps:

a) pulverizing: weighing the plant raw materials according to parts by weight respectively and pulverizing them;

b) soaking: the pulverized plant raw materials from step a) are placed into a sealed container, and then water is added thereto in a solid/liquid ratio of 1:4 by weight, and the resultant is soaked for 2-5 h;

c) ultrasonic extracting: the plant raw materials and soak solution obtained from step b) are extracted in an ultrasonic tank, where the temperature was raised from a room temperature to no greater than 40°C, and the extraction time was 50-60 min;

d) filtrating and separating: the mixed solution obtained from step c) is filtered to obtain a filtrate and a residue, the residue is subjected to centrifugal filtration to obtain a secondary filtrate, and both the filtrates are combined into a final filtrate;

e) adjusting pH: the final filtrate is adjusted to having a pH of 7.5-7.7;

f) concentrating: the final filtrate is concentrated to a concentrated product having a content of 75-80%;

g) adding a plant extract: the plant extract is weighted according to parts by weight, added into the concentrated product and mixed homogeneously; and

h) drying: the mixture obtained from step g) is dried to provide a dry final product.

In step c), the stirring rate in the ultrasonic tank is 1500 rpm, the ultrasonic power is 1500 W, and the ultrasonic interval is 1.5 min.

In said step h), vacuum drying or microwave drying may be used. In the case of vacuum drying, the influence of air on the product is reduced, the drying temperature is low, the drying rate is fast, and the contacting chance between the product and air is decreased, as well as contamination and oxidative deterioration are avoided, and the product is easily pulverized.

In the case of microwave drying, the material to be dried is radiated by a microwave generator, and when the microwave is irradiated to the inside of the material, polar molecules such as water move synchronously with the microwave frequency, so that frictional heat is generated instantaneously in the material, and thus the temperatures on the surface and inside of the material are increased simultaneously, then a large amount of the water molecules escape outside, hereby the drying of the material is achieved. The microwave drying has a high drying rate, short drying time, and uniform heating, thus it is very suitable for drying and sterilizing the raw materials of Chinese traditional medicines. The volatile and aromatic substances in the raw materials are less lost, because the drying time is 10- or 100-fold of that of conventional heating.

The dry final product made from the plant extract composition according to the present invention may be pulverized and prepared into a desired formulation such as capsule, tablet, powder, or granule, optionally with the addition of suitable adjuvants or carriers. Each of these formulations may be prepared according to conventional methods in the pharmaceutical field.

The plant extract composition of this invention is prepared by employing ultrasonic to extract the plant raw materials, and during preparing, no chemical reagent is used, thus natural active ingredients of the plants are remained so that various natural flavones, soaps, phospholipids and various enzymes are contained therein. After entering the blood vessels, the plant extract composition of the invention can increase the level of HDL in blood, and efficiently and quickly decompose and dissolve the lipids and blood deposits in blood. Simultaneously, it can dilate and soften the blood vessels, restore the tension and elasticity of the blood vessels, and repair the blood vessels to reduce the vascular resistance; and can also decrease the blood viscosity, purify the blood, and eliminate hazardous substances, rubbish and toxins in the blood vessels from the body, actually functioning as a scavenger of blood vessels.

### DETAILED DESCRIPTION OF THE INVENTION

Embodiment 1

A plant extract composition functioning as a scavenger of blood vessels was prepared according to the following steps:

a) pulverizing: the plant raw materials were weighted according to parts by weight, respectively: 10 parts of Folium Isatidis, 35 parts of Semen Cassiae, 10 parts of Flos Inulae, 15 parts of Flos Carthami, 10 parts of Flos Dendranthemae, and 50 parts of Folium Apocyni Veneti, and pulverized;

b) soaking: the pulverized plant raw materials in step a) were placed into a sealed container, and then water was added thereto in a solid/liquid ratio of 1:4 by weight, and the resultant was soaked for 3 hours;

c) ultrasonic extracting: the plant raw materials and soak solution obtained from step b) were extracted in an ultrasonic tank, where the temperature was raised from a room temperature to 30°C, and the extraction time was 60 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to having a pH of 7.5;

f) concentrating: the final filtrate was concentrated to a concentrated product having a content of 75%;

g) adding a plant extract: the plant extract was weighted according to parts by weight: 40 parts of Ginkgo flavone, 20 parts of emodin, 30 parts of crataegin, 15 parts of gypenoside, and 60 parts of Panax notoginseng saponins, and added into the concentrated product and mixed homogeneously; and

h) drying: the mixture obtained from step g) was dried under vacuum to provide a dry final product.

Embodiment 2

A plant extract composition functioning as a scavenger of blood vessels was prepared according to the following steps:

a) pulverizing: the plant raw materials were weighted according to parts by weight, respectively: 30 parts of Folium Isatidis, 15 parts of Semen Cassiae, 20 parts of Flos Inulae, 30 parts of Flos Carthami, 30 parts of Flos Dendranthemae, and 20 parts of Folium Apocyni Veneti, and pulverized ;

b) soaking: the pulverized plant raw materials in step a) were placed into a sealed container, and then water was added thereto in a solid/liquid ratio of 1:4 by weight, and the resultant was soaked for 2 hours;

c) ultrasonic extracting: the plant raw materials and soak solution obtained from step b) were extracted in an ultrasonic tank, where the temperature was raised from a room temperature to 35°C, and the extraction time was 50 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to having a pH of 7.6;

f) concentrating: the final filtrate was concentrated to a concentrated product having a content of 80%;

g) adding a plant extract: the plant extract was weighted according to parts by weight: 20 parts of Ginkgo flavone, 35 parts of emodin, 50 parts of crataegin, 55 parts of gypenoside, and 20 parts of Panax notoginseng saponins, and added into the concentrated product and mixed homogeneously; and

h) drying: the mixture obtained from step g) was dried under vacuum to provide a dry final product.

Embodiment 3

A plant extract composition functioning as a scavenger of blood vessels was prepared according to the following steps:

a) pulverizing: the plant raw materials were weighted according to parts by weight, respectively: 15 parts of Folium Isatidis, 30 parts of Semen Cassiae, 12 parts of Flos Inulae, 30 parts of Flos Carthami, 25 parts of Flos Dendranthemae, and 40 parts of Folium Apocyni Veneti, and pulverized, additionally, 10 parts of black Auricularia was weighted and pulverized;

b) soaking: the pulverized plant raw materials and black Auricularia in step a) were placed into a sealed container, and then water was added thereto in a solid/liquid ratio of 1:4 by weight, and the resultant was soaked for 4 hours;

c) ultrasonic extracting: the plant raw materials and soak solution obtained from step b) were extracted in an ultrasonic tank, where the temperature was raised from a room temperature to 40°C, and the extraction time was 50 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to having a pH of 7.7;

f) concentrating: the final filtrate was concentrated to a concentrated product having a content of 80%;

g) addiing a plant extract: the plant extract was weighted according to parts by weight: 25 parts of Ginkgo flavone, 25 parts of emodin, 30 parts of crataegin, 20 parts of gypenoside, and 50 parts of Panax notoginseng saponins, as well as 30 parts of lecithin and 20 parts of solid acetic acid, and added into the concentrated product and mixed homogeneously; and

h) drying: the mixture obtained from step g) was dried under microwave to provide a dry final product.

Embodiment 4

A plant extract composition functioning as a scavenger of blood vessels was prepared according to the following steps:

a) pulverizing: the plant raw materials were weighted according to parts by weight, respectively: 20 parts of Folium Isatidis, 25 parts of Semen Cassiae, 15 parts of Flos Inulae, 25 parts of Flos Carthami, 20 parts of Flos Dendranthemae, and 30 parts of Folium Apocyni Veneti, and pulverized, additionally, 20 parts of black Auricularia was weighted and pulverized;

b) soaking: the pulverized plant raw materials and black Auricularia in step a) were placed into a sealed container, and then water was added thereto in a solid/liquid ratio of 1:4 by weight, and the resultant was soaked for 3 hours;

c) ultrasonic extracting: the plant raw materials and soak solution obtained from step b) were extracted in an ultrasonic tank, where the temperature was raised from a room temperature to 30°C, and the extraction time was 60 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to having a pH of 7.5;

f) concentrating: the final filtrate was concentrated to a concentrated product having a content of 80%;

g) adding a plant extract: the plant extract was weighted according to parts by weight: 30 parts of Ginkgo flavone, 30 parts of emodin, 35 parts of crataegin, 25 parts of gypenoside, and 40 parts of Panax notoginseng saponins, as well as 20 parts of lecithin and 80 parts of acetic acid solid, and added into the concentrated product and mixed homogeneously; and

h) drying: the mixture obtained from step g) was dried under microwave to provide a dry final product.

Embodiment 5

A plant extract composition functioning as a scavenger of blood vessels was prepared according to the following steps:

a) pulverizing: the plant raw materials were weighted according to parts by weight, respectively: 25 parts of Folium Isatidis, 20 parts of Semen Cassiae, 18 parts of Flos Inulae, 20 parts of Flos Carthami, 15 parts of Flos Dendranthemae, and 30 parts of Folium Apocyni Veneti, and pulverized;

b) soaking: the pulverized plant raw materials in step a) were placed into a sealed container, and then water was added thereto in a solid/liquid ratio of 1:4 by weight, and the resultant was soaked for 5 hours;

c) ultrasonic extracting: the plant raw materials and soak solution obtained from step b) were extracted in an ultrasonic tank, where the temperature was raised from a room temperature to 40°C, and the extraction time was 50 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to have a pH of 7.6;

f) concentrating: the final filtrate was concentrated to a concentrated product having a content of 80%;

g) adding a plant extract: the plant extract was weighted according to parts by weight: 35 parts of Ginkgo flavone, 35 parts of emodin, 40 parts of crataegin, 40 parts of gypenoside, and 30 parts of Panax notoginseng saponins, and added into the concentrated product and mixed homogeneously; and

h) drying: the mixture obtained from step g) was dried under vacuum to provide a dry final product.

Embodiment 6

A plant extract composition functioning as a scavenger of blood vessels was prepared according to the following steps:

a) pulverizing: the plant raw materials were weighted according to parts by weight, respectively: 20 parts of Folium Isatidis, 25 parts of Semen Cassiae, 16 parts of Flos Inulae, 25 parts of Flos Carthami, 20 parts of Flos Dendranthemae, and 35 parts of Folium Apocyni Veneti, and pulverized, and then 30 parts of black Auricularia was weighted and pulverized;

b) soaking: the pulverized plant raw materials in step a) were placed into a sealed container, and then water was added thereto in a solid/liquid ratio of 1:4 by weight, and they were soaked for 3 hours;

c) ultrasonic extracting: the plant raw materials and soak solution obtained in step b) were extracted in a ultrasonic tank, where the temperature was raised from a room temperature to 40°C, and the extraction time was 50 minutes;

d) filtrating and separating: the mixed solution obtained from step c) was filtered to obtain a filtrate and a residue, the residue was subjected to centrifugal filtration to obtain a secondary filtrate, and both the filtrates were combined into a final filtrate;

e) adjusting pH: the final filtrate was adjusted to have a pH of 7.7;

f) concentrating: the final filtrate was concentrated to a concentrated product having a content of 80%;

g) adding a plant extract: the plant extract was weighted according to parts by weight: 30 parts of Ginkgo flavone, 30 parts of emodin, 35 parts of crataegin, 30 parts of gypenoside, and 40 parts of Panax notoginseng saponins, as well as 10 parts of lecithin and 60 parts of solid acetic acid, and added into the concentrated product and mixed homogeneously; and

h) drying: the mixture obtained from step g) was dried under microwave to provide a dry final product.

Embodiment 7: Animal Experiment

Experimental method: 10 mice were randomly divided into two groups of A and B with 5 for each. Group A was fed with a conventional feed added with the dry final product of the composition prepared in embodiment 5 of this invention. Group B as a comparative group was fed with a conventional feed.

The mice from each of groups A and B were fed three times per day. And they were observed for 30 days.

Experimental results: during the 30-day feeding, the mice from group A appeared more active with a relatively high sensitivity, and the mice from group B appeared less active.

It was found from the lipid analysis post 30 days that: HDL in the blood of the mice from group A was greater than that from group B by 32.53% on average, triglyceride in the blood of those from group A was less than that from group B by 27.16% on average, and the blood viscosity of those from group A was less than that from group B by 25%. Moreover, it was found from dissection that the elasticity of blood vessels was normal for the mice from group A, and for those from group B, the degree of vascular sclerosis was increased by above 15% and the thickness of blood vessel walls was increased by 13.2%. No damage to the liver or kidney was found in all of the 5 mice from group A after dissection.

Embodiment 8: Clinical Trials

Experimental method: 5 patients with hypertension, 5 patients with stroke and paralysis, and 5 patients with angina. Each of the patients was administered with the dry final product powder of the composition prepared in embodiment 4 of this invention, and took the medicine three times a day by 3 g for each with warm water for 8 courses of treatment in total, in which 30 days was deemed as one course of treatment.

Experimental results:

Patients with hypertension

5 patients with hypertension had a diastolic pressure of above 180 and a systolic pressure of above 110, with the chief complaints of dizziness, tinnitus, chest distress, short of breath, and insomnia, and with partial blockage appeared in the skull Doppler.

After 8 courses of treatment using the composition of this invention, the diastolic pressures of 3 patients therein were reduced to below 140, the systolic pressures thereof were around 100, the chief complaints disappeared, no vascular abnormality was shown in the skull Doppler, and the electrocardiogram showed that the blood supply for the heart and brain was normal; the diastolic pressure of another one patient was 150, the systolic pressure was 100, the chief complaints essentially disappeared, no vascular abnormality was shown in the skull Doppler, and the electrocardiogram showed that the blood supply for the heart and brain was normal; and it was ineffective for the remained one.

Patients with stroke

5 patients had the symptoms of dottiness, eye-mouth deviation, and paralysis in bed, with one of them lying in bed for 1 year.

After 8 courses of treatment using the composition of this invention, 4 patients of them could get out of bed by themselves, walk slowly with a stick, and do some simple housekeeping independently; and 1 patient therein began to be sober-minded, could speak basically, and could raise both arms and legs by himself/herself.

Patients with angina

5 patients had the symptoms of palpitation, short of breath, choking, irregular attacks of chest pain, panting on walking, and weakness of legs, with a severe shortage of blood supply appeared in the electrocardiogram.

One of the 5 patients was 75 years old, and thus was administered three times a day with 1.5 g for each for 4 courses of treatment, and the remained patients were normally administered for 8 courses of treatment. Among four (including the one of 75 years old above) of these patients, the symptoms substantially disappeared, and the electrocardiograms showed that the blood supply was normal; and in one of the five patients, the symptom of choking disappeared, the attack duration and frequency of chest pain were reduced, and the electrocardiogram showed that the blood supply was basically normal.

## Claims

1. A plant extract composition functioning as a scavenger of blood vessels, mainly composed of plant raw materials and an extract, wherein the plant raw materials comprise the following components according to parts by weight: 10-30 parts of Folium Isatidis, 15-35 parts of Semen Cassiae, 10-20 parts of Flos Inulae, 15-30 parts of Flos Carthami, 10-30 parts of Flos Dendranthemae, and 20-50 parts of Folium Apocyni Veneti; and the extract comprises the following components according to parts by weight: 20-40 parts of Ginkgo flavone, 20-35 parts of emodin, 30-50 parts of crataegin, 15-55 parts of gypenoside, and 20-60 parts of Panax notoginseng saponins.

2. The plant extract composition of claim 1, wherein the plant raw materials comprise the following components according to parts by weight: 15-25 parts of Folium Isatidis, 20-30 parts of Semen Cassiae, 12-18 parts of Flos Inulae, 20-30 parts of Flos Carthami, 15-25 parts of Flos Dendranthemae, and 30-40 parts of Folium Apocyni Veneti; and the extract comprises the following components according to parts by weight: 25-35 parts of Ginkgo flavone, 25-35 parts of emodin, 30-40 parts of crataegin, 20-40 parts of gypenoside, and 30-50 parts of Panax notoginseng saponins.

3. The plant extract composition of claim 1, further comprising the following raw materials according to parts by weight: 10-30 parts of lecithin, 10-30 parts of black Auricularia, and 20-80 parts of solid acetic acid.

4. The plant extract composition of claim 2, further comprising the following raw materials according to parts by weight: 10-30 parts of lecithin, 10-30 parts of black Auricularia, and 20-80 parts of solid acetic acid.

5. The plant extract composition of any one of claims 1-4, wherein the formulation of the plant extract composition is capsule, tablet, powder, or granule.

6. A method for preparing the plant extract composition of any one of claims 1-4, comprising the following steps:
a) pulverizing: weighing the plant raw materials according to parts by weight respectively and pulverizing them;
b) soaking: the pulverized plant raw materials from step a) are placed into a sealed container, and then water is added thereto in a solid/liquid ratio of 1:4 by weight, and the resultant is soaked for 2-5 hours;
c) ultrasonic extracting: the plant raw materials and soak solution obtained from step b) are extracted in an ultrasonic tank, where the temperature was raised from a room temperature to no greater than 40°C, and the extraction time was 50-60 minutes;
d) filtrating and separating: the mixed solution obtained from step c) is filtered to obtain a filtrate and a residue, the residue is subjected to centrifugal filtration to obtain a secondary filtrate, and both the filtrates are combined into a final filtrate;
e) adjusting pH: the final filtrate is adjusted to having a pH of 7.5-7.7;
f) concentrating: the final filtrate is concentrated to a concentrated product having a content of 75-80%;
g) adding a plant extract: the plant extract is weighted according to parts by weight, added into the concentrated product and mixed homogeneously; and
h) drying: the mixture obtained from step g) is dried to provide a dry final product.

7. The method of claim 6, wherein in the step c), the stirring rate in the ultrasonic tank is 1500 rpm, the ultrasonic power is 1500 W, and the ultrasonic interval is 1.5 minutes.

8. The method of claims 6 or 7, wherein in the step h), vacuum drying or microwave drying is used.

## Patentansprüche

1. Zusammensetzung des Pflanzenextraktesmit der vaskulären Reinigerfunktion, vor allem aus Pflanzenmaterial und Extrakt bestehend" wobei das Pflanzen material folgendeBestandteile von den folgenden Gewichtsteilen enthält:Herba Taching 10-30 Portionen,Semen Cassiae Torae 15-35 Portionen, Inula-Blume 10-20 Portionen, Carthamus Tinctorious 15-30 Portionen, Chrysantheme 10-30 Portionen, Bluish Dogbane 20-50 Portionen;und wobei der Extrakt folgendeBestandteile von den folgenden Gewichtsteilen enthält:Ginkgo Flavonoids 20-40 Portionen, Emodin 20-35 Portionen, Cralaegin 30-50 Portionen, Gypenosides 15-55 Portionen, Panax Notoginsenosides 20-60 Portionen.

2. Zusammensetzung des Pflanzenextraktes nach Anspruch 1, **dadurch gekennzeichnet, dass** das Pflanzenmaterial folgende Gewichtsteile hat: Herba Taching 15-25 Portionen, Semen Cassiae Torae 20-30 Portionen, Inula-Blume 12-18 Portionen, Carthamus Tinctorious 20-30 Portionen, Chrysantheme 15-25 Portionen, Bluish Dogbane 30-40 Portionen; und wobei der Extrakt folgende Gewichtsteile hat: Ginkgo Flavonoids 25-35 Portionen, Emodin 25-35 Portionen, Cralaegin 30-40 Portionen, Gypenosides 20-40 Portionen, Panax Notoginsenosides 30-50 Portionen.

3. Zusammensetzung des Pflanzenextraktes nach Anspruch 1, **dadurch gekennzeichnet, dass** Materialien von folgenden Gewichtsteilen noch enthalten sind: Lecithin 10-30 Portionen, und schwarze Pilze 10-30 Portionen, solides Acetat 20-80 Portionen.

4. Zusammensetzung des Pflanzenextraktes nach Anspruch 2, **dadurch gekennzeichnet, dass** Materialien von folgenden Gewichtsteilen noch enthalten sind: Lecithin 10-30 Portionen, und schwarze Pilze 10-30 Portionen, solides Acetat 20-80 Portionen.

5. Zusammensetzung des Pflanzenextraktes nach einem von Ansprüchen 1 bis 4, **dadurch gekennzeichnet, dass** Zusammensetzung in Form der Kapsel, der Tablette, des Pulvers und des Granulatessein kann.

6. Zusammensetzung des Pflanzenextraktes nach einem von Ansprüchen 1 bis 4, aufweisende folgende Schritte:
a) Pulverisieren, wobei die Pflanzenmaterialien zu den obenerwähnten Gewichtsteilen genommen und pulverisiert werden;
b) Einweichen, wobei die im Schritt a) pulverisiertenPflanzenmaterialien in einen geschlossenen Behälter gelegt werden, und wobei das Wasser nach einem Fest-Flüssig-Gewichtsverhältnis von 1:4 in den Behälter gefüllt wird, und wobeidas Einweichen 2-5 Stunden dauert;
c) Ultraschall-Extraktion, wobei die durch den Schritt b) erhaltenen Pflanzenmaterialien und die Einweich-Flüssigkeit imUltraschallbehälter extrakiert werden, und wobei die Temperatur sich von Raumtemperatur auf die Temperatur von nicht über 40° C erhöht, und wobei die Extraktion 50-60 Minuten dauert;
d) Trennen durch Filtration, wobei die durch den Schritt c) erhaltene Mischungsflüssigkeit filtriertwird, und wobei das Filtrat und der Filterrückstand erhalten werden, und wobei das zweite Filtrat durch die Zentrifugationsfiltrierung des Filtratrückstands erhalten wird, und wobei die beiden Filtrate kombiniert werden und das Filtrat entsteht;
e) pH-Wert einstellen, wobeider pH-Wert des Filtrates auf 7,5 bis 7,7 eingestellt wird;
f) Konzentration, und wobei das Filtrat auf das Konzentratmit einer Konzentration von 75-80% konzentriert wird.
g) Die obenerwähnten Pflanzenextrakten werden zugesetzt, die Extrakten zu obenerwähnten Gewichtsteilen werden genommen und im Konzentrat hinzugefügt, gleichmäßig gemischt.
h) Trocknen, die im Schritt g) erhaltene Mischung wird getrocknet, um das Endprodukt zu ergeben.

7. Herstellungsverfahren nach Anspruch 6, **dadurch gekennzeichnet, dass** im Schritt c) die Rührdrehzahlim Ultraschallbehälter1500/min beträgt, und wobei die Ultraschall-Leistung 1500W beträgt, und wobei die Pause des Ultraschalls 1,5 Minuten dauert.

8. Herstellungsverfahren nach Anspruch 6 oder 7, **dadurch gekennzeichnet, dass** im Schritt h) das Unterdruck-Trocknen oder das Mikrowellen-Trocknen eingesetzt werden können.

## Revendications

1. Une extrait du plante a la fonction de nettoyeur du vaisseau sanguin. combinaison entre de matière première plante et d'extrait du plante. La matière première plante compose au poids suivants: le folium isatidis 10-30 parts, la semem cassiae 15-35 parts, le fleur inula 10-20 parts, la carthame des teinturiers 15-30 parts, le chrysanthèmemum 10-30 parts, l'apocynum venetum 20-50 parts; l'extrait du plante compose au poids suivants: la flavone ginkgo 20-40 parts, l'émodine 20-35 parts, l'élément d'aubépine 30-50 parts, le gypenosides GP 15-55 parts, le triterpènes notoginseng 20-60 parts.

2. selon la reclamation 1, la combinaison de l'extrait du plante, caracté rise par la composition au poids de les matière première plantes suivante: le folium isatidis 15-25 parts, la semem cassiae 20-30 parts, le fleur inula 12-18 parts, la carthame des teinturiers 20-30 parts, le chrysanthèmemum 15-25 parts, l'apocynum venetum 30-40 parts; l'extrait du plante compose au poids suivants: la flavone ginkgo 25-35 parts, l'émodine 25-35 parts, l'élément d'aubépine 30-40 parts, le gypenosides GP 20-40 parts, le triterpènes notoginseng 30-50 parts.

3. selon la reclamation 1, la combinaison de l'extrait du plante, caracté rise par la composition au poids de la matière première suivante: la lecithine 10-30 parts, le champignon noir 10-30 parts, l'acide acétique la solide 20-80 parts.

4. selon la reclamation 2, la combinaison de l'extrait du plante, caracté rise par la composition au poids de la matière première suivante: la lecithine 10-30 parts, le champignon noir 10-30 parts, l'acide acétique la solide 20-80 parts.

5. n'importe quelle combinaison parmi la reclamation 1-4, **caractérise par** la type pharmaceutique peuvent être la type suivant : l'agent capsule, la pastille, la poudre, la particule.

6. n'importe quelle combinaison parmi la reclamation 1-4, qui comporte l'étapes suivante:
a) Réduire en poudre, prenez la matière première par la composition au poids que pesez séparément pour réduire en poudre ;
b) Tremper, dans l'étape a) mettre la matière première en poudre dans un récipient fermé, ajouter l'eau dedans par la proportion de solide et liquide au poids par 1: 4. tremper les pendant de deux heures à cinq heures;
c) Prélèvement ultrasonique, la matière première et la liquide que on obtenir par l'étape b) prélevent dans un pot ultrasonique. réchauffer les temperature de normal jusqu'à quarante degrés Celsius. la durée prélèvement est entre 50-60 minutes;
d) Filtrer et Dissocier, le filtrat et le sediment qu'on obtenir par l'étape c), le sediment avoir été par filtrer centrifuge, qui obtenir la filtrate secondaire. mettre le prmier et la seconde filtrate tout ensemble pour la filtrate uni;
e) Régler la PH, règle la filtrate uni entre 7.5PH-7.7 PH;
f) Concentration, concentrer la filtrate uni jusqu'à 70%-80%;
g) Mettre ensemble l'extrait du plante pesant au poids et la concentration pour f), bien les mélanger ;
h) Séchage , sécher laquelle après la étape g) pour la produit séché fini.

7. selon la méthode de preparation par la reclamation 6, son caracté ristique concerne l'étape c), la vitesse de mélanger dans un pot ultrasonique doit être 1500/minute, la puissance doit être 1500W, la fréquence doit être une et demi minutes.

8. selon la méthode de preparation par la reclamation 6 ou 7, son caracté ristique concerne l'étape h), peuvent utiliser la méthode de séchage pression or séchage micro.
